# EUROPEAN PATENT SPECIFICATION

(11) **EP 3 693 053 B1**
(45) Date of publication and mention of the grant of the patent: **05.01.2022**
(21) Application number: 19383020.5
(22) Date of filing: 20.11.2019
(51) Int. Cl.: A61N 1/04, A61N 1/36

(54) **AURICULAR NEUROSTIMULATION DEVICE AND SYSTEM**
AURIKULÄRE NEUROSTIMULATIONSVORRICHTUNG UND SYSTEM
DISPOSITIF DE NEUROSTIMULATION AURICULAIRE ET SYSTÈME

(43) Date of publication of application: 12.08.2020
(73) Proprietor: XanaStim Sàrl, 1066 Epalinges (CH)
(72) Inventor: LARRAYA, Iñaki, 31600 Burlada (ES); LOPEZ FERNANDEZ, Miguel, 33391 Gijón (ES); BERMEJO, Pedro Emilio, 28914 Leganés (ES); GARCIA DE GURTUBAY, Iñaki, 31190 Cizur Menor (ES); FARRE, Alberto, 25660 Alcoletge (ES)
(74) Representative: Hernandez Lehmann, Aurelio

(56) References cited:
- WO-A1-2019/005774
- WO-A1-2019/014250
- WO-A1-2019/028000
- CN-A- 109 718 470
- DE-A1-102015 012 684
- DE-B3-102015 007 215
- US-A1- 2012 035 680
- US-A1- 2015 018 925
- US-A1- 2015 165 195

## Description

### Field of Invention

The present invention relates to a connected auricular neurostimulation device. The invention further refers to an auricular neurostimulation system comprising an auricular neurostimulation device having a higher efficiency and able to be personalized, adapted to each user and to each user's requirements. The invention further relates to a method for the operation of such a system.

### Background Prior Art

The vagus nerve (VN) is the longest of the twelve cranial nerves. The VN connects our brainstem to the body, allowing the brain to receive control and monitor several body functions automatically. The VN is the main nerve of the parasympathetic division of the autonomic nervous system (ANS) and forms a sort of electrical circuit that regulates heart rate, gastrointestinal motility and secretion, pancreatic endocrine and exocrine secretion, hepatic glucose production, and other visceral functions.

The VN comprises two bilateral nerves, both of which root in the brain stem and branch out through the neck and down each side of the body, across the abdomen and to the main organs. Besides giving some output to various organs, the VN comprises between 80% and 90% of afferent nerves mostly conveying sensory information about the state of the body's organs to the central nervous system playing a key role in the neuroendocrine-immune axis to maintain homeostasis through its afferents to the hypothalamic pituitary adrenal axis and the central ANS, and through its cholinergic afferents.

Due to this wide influence on multiple systems and its important role in maintaining homeostasis, stimulating the VN to modulate the function of related organs has long drawn the attention of investigators. As a slow-acting therapy, the US Food and Drug Administration have approved invasive cervical vagus nerve stimulation (VNS) for managing treatment-refractory epilepsy in 1997 and for chronic treatment-resistant depression in 2005.

The first medical device to treat pathologies linked with the vagus nerve was developed by the company Cyberonics Inc., currently Livanova Inc. Other known vagus nerve stimulation devices now approved or being assessed at present for medical use are for example the following:
- Maestro, an invasive VNS device from EnteroMedics (now ReShape Lifesciences), for obesity.
- Vivistim, from MicroTransponder, is an implantable VNS device for stroke victims. The company is also testing Serenity, which uses an implantable device together with a tone-producing headset for perfect symmetry of the electrochemical processes in the two half-waves of the pulses should be sought tinnitus.
- SetPoint Medical, an invasive procedure for rheumatoid arthritis and other inflammatory diseases.
- CardioFit from BioControl Medical, a VNS for congestive heart failure.

However, these known vagus nerve stimulation devices present several problems, such as surgical risks, technical challenges, and potential side effects, which have therefore limited their application. To overcome such barriers of applying invasive vagus nerve stimulation devices (VNS), non-invasive transcutaneous vagus nerve stimulation (tVNS) methods have been developed instead. Currently, there are two known main ways of applying tVNS: one is to superficially apply stimulation on the cervical nerve using a specially designed device, such as GammaCore, and the other one is to stimulate the Auricular Branch of Vagus Nerve (ABVN) on the ear.

Neural anatomy has shown that the auricular branch of the vagus nerve (ABVN) projects to the ipsilateral nucleus tractus solitarii (NTS), locus coeruleus (LC) and contralateral parabrachial nucleus (PBN). From the contralateral parabrachial nucleus, it propagates to various brain regions as hypothalamus (Hth), amygdala (Am) and nucleus accumbens (TA) (Frango E. et, al., 2015, Badran B, et. al, 2017).

The ABVN is the target of a form of noninvasive brain stimulation, known as auricular vagus nerve stimulation (aVNS). aVNS has been used to treat disorders, such as depression, anxiety, schizophrenia, autism spectrum, epilepsy (Rong P. et al., 2014, Stefan H. et al., 2012), migraine (Silberstein SD, et al., 2016), chronic tinnitus (Shim HJ et al., 2017), pre-diabetes (Huang F, et al, 2014), metabolic syndrome (Kaniusas et al., 2019), rehabilitation after ischemic stroke (Baig SS, et al. 2019), ventricular arrhythmias (Nasi-Er BG, et. al, 2019), among others, as well as to boost associative memory (Jacobs HI, et al. 2015), what has been proposed to help patients with Alzheimer's disease and other dementia types (Kaczmarczyk R, et. al 2017, Cai L, et.al 2019).

Some known devices in the state of the art electro stimulate areas of the ear with presence of ABVN: Nemos^{®} from Cerbomed, Parasym^{®} from Parasym LTD, Nervana^{®} from Nervana LLC and Net 1000^{®} from Auri-Stim Medical, amongst others.

However, the stimulation of the ear in the referred devices known in the prior art, is not selective for ABVN, since these areas are supplied not only by ABVN, but also by the auriculotemporal, glossopharyngeal, facial and spinal cervical nerves, among others. The cymba conchae is the only outer ear zone regarded to be exclusively innervated by ABVN. According to Peuker and Filler, 2002, the other ear areas supplied by ABVN are the anti-helix (73% with ABVN), the cavity of conchae and tragus (45% with ABVN), the crus of helix (20% with ABVN) and the crura of anti-helix (9% with ABVN).

US 2012/0035680 (A1) and WO 2019/014250 (A1) describe a device that electrically stimulates afferent fibres of the auricular branch of the vagus nerve taking into account the user's pulmonary activity (Respiratory-gated Auricular Vagal Afferent Nerve Stimulation - RAVANS). The control of the stimulation is carried out using an electrical circuit connected on one side to two electrodes that apply the stimulation voltage and on the other side to a respiratory belt with a strain gage, a nasal air flow detector (US 2012/0035680 (A1)) or a pulse sensor configured to measure blood pressure (WO 2019/014250 (A1)) that send electrical signals associated with the pulmonary activity. The fact that the user has to carry and connect several devices significantly reduces the usability of the device.

The device incorporates two electrodes attached to afferent fiber zones of the auricular branch of the vagus nerve wherein the electrodes are described as *"small discs made from conductive material and attached to the patient using an adhesive band. Similarly, pre-gelled circular or spherical silver*/*silver chloride electrodes can be used".*

WO 2019/005774 (A1) describes a device for transcutaneous electrical stimulation of peripheral nerves including the auricular branch of the vagus nerve. The device comprises a control unit and a housing to be placed on or in the ear, with two electrodes connected to the control unit, which can modulate the electrical current applied to the electrodes. The control unit or the housing can be fitted with a sensor to measure the user's physiological parameters, on the basis of which the stimulation parameters can be adjusted. These parameters include heart rate variability (HRV) and oxygen saturation.

This document discloses a pair of electrodes that are located *"on an external periphery of an cylindrical interface member having a C-shaped cross-section that engages a target portion of a patient's ear".*

The device described uses therefore a standard geometry housing for the electrode holder, which does not ensure that the contact with the area to be stimulated is good enough due to the great anatomical variability of human ears. Besides, the disclosed device uses standard electrode geometry and due again to the anatomical variability this does not ensure that the contact is sufficiently wide and of good quality. Both of the above characteristics make the stimulation less effective and comfortable. On the other hand, the document does not mention whether the stimulation is anodic or cathodic. The addition of a delay between the stimulation pulse and the reversion pulse is also not mentioned.

EP3100764 (A1) shows an neurostimulation device from Cerbomed that stimulates the nerve ramifications only of cymba by means of two electrodes. However, these two electrodes are small as they must fit in the cymba reserving a space between them to avoid short circuit. On the other hand they are located on a standard support that has limitations to adapt to the variable geometry of the cymba of the users, reason why in many cases the contact of the electrodes with the cymba is very poor. Both characteristics make the stimulated area of the cymba very small and the general efficiency of the device is reduced. In addition, all the electronics of the device are external (outside the ear cavity), which implies significant needs for wiring, connections, etc. that make the device as a whole very bulky and uncomfortable to use.

PCT/EP2015/001279 discloses a stimulation pattern of a neurostimulator similar to that of EP3100764 (A1). It is a trapezoidal, asymmetric biphasic wave starting with a positive pulse (anodic stimulation). It is estimated that the depolarization that occurs with an anodic stimulation is roughly one-seventh to one-third that of the depolarization with cathodic stimulation (the waveform begins with a negative pulse).

These known devices use electrical current as a means of stimulating nerve endings. However, an anatomical study performed by the applicants found that nerve endings in the auricular region correspond to mechanoreceptors and thermoreceptors that respond to mechanical and thermal stimuli, respectively. Therefore, it is also possible to activate these nerve endings with mechanical stimuli such as fine touch or vibration, thus resulting in more efficient devices, easier to adapt to the users. Furthermore, known devices stimulate nerves in the ear in areas where there is no high concentration or no concentration at all of ABVN, thus resulting in inefficient devices. Moreover, personalization to adapt correctly these devices so they fit to each user is key, and is not provided by the devices known in the state of the art.

In the published document reviews "Current Directions in the Auricular Vagus Nerve Stimulation I - A Physiological Perspective, II - An Engineering Perspective" (Kanlusas et al., August 2019) it is disclosed that the electrical stimulation of the auricular vagus nerve (aVNS) is an emerging technology in the field of bioelectronic medicine with applications in therapy, the modulation of the afferent vagus nerve affecting a large number of physiological processes and bodily states associated with information transfer between the brain and body. The aVNS is seen as a therapeutic tool for current research lines, highly relevant for the promising aVNS technology to reach the patient. Therefore, developing a device stimulating the aVNS the more efficiently possible and personalized for each patient is a need in the current developments for this emerging technology.

The object of the invention is a wearable connected auricular neurostimulation device that stimulates the nerve ramifications of cymba and cavum conchae having a higher efficiency, being comfortable to wear and allowing to be personalized such that it can adapt to each user and to each user's requirements.

The invention also aims at other objects and at the solution of other problems as will appear in the rest of the present description.

### Summary of the invention

In view of the foregoing prior art, an object of the present invention is to provide, according to a first aspect, an auricular neurostimulation device wearable by a user and configured to stimulate the Auricular Branch of Vagus Nerve (ABVN) on the user's ear; the device comprising at least one electrode designed to be located in the cymba and another electrode in the cavum conchae. The cymba electrode uses the entire area of the cymba to stimulate the ABVN present in the zone when a voltage difference is applied to it with respect to the cavum conchae electrode.

Preferably, the electrodes are made of graphene, biocompatible metals such as titanium, nickel titanium (nitinol), platinum, platinum-iridium, non-toxic metals as gold, conductive biocompatible inks for 3D printing or flexible conductive biocompatible polymers that adapt to the anatomy of the stimulation zone, therefore providing good comfort and perfect adaptation to the patient's ear.

Typically, the auricular neurostimulation device further comprises an earmold where the electrodes are arranged, the earmold being customized to the user's anatomy to achieve good contact between the electrodes and the stimulation zones.

Preferably, the auricular neurostimulation device of the invention further comprises a photoplethysmographic or biosensor estimating the amount of hemoglobin and oxyhemoglobin circulating through the most superficial capillary vessels of the ear of the patient or user, these data being used to calculate the heart rate, the heart rate variability (HRV) and to detect the breathing phase (exhalation or inhalation) of the user.

Typically, the photoplethysmographic or biosensor is configured to detect a low heart rate of the user (bradycardia), in which case the stimulation of the device will be stopped to avoid any cardiological risk.

Preferably, in the auricular neurostimulation device of the invention, the stimulation done by it is synchronized with the exhalation-breathing phase of the user.

The auricular neurostimulation device of the invention typically implements three types of stimulation protocols: BEAT, BFS and EVANS, with variable stimulation parameters in all of them.

Preferably, the stimulation protocols are based on a waveform of rectangular, biphasic, symmetric and with a delay between the negative and positive pulse.

Typically, the stimulation protocol is of the BEAT type, consisting of the continuous application of bursts of pulses.

In one preferred embodiment, the stimulation protocol is a BFS (Breathing Focused on Stimulation) type combining stimulation moments with standstill moments, so the user breathes in during the stop time and breathes out during the stimulation. This type of protocol makes it easier for the user to focus his attention on stimulation and thus benefit from the meditation effect. It also allows the duration of breathing to be extended and the benefits of slow breathing to be added. In this way, the protocol BFS adds to the beneficial effect of relaxation, meditation and slow breathing.

In another embodiment, the stimulation protocol is a EVANS (Exhalation Vagus Auricular Nerve Stimulation) type, in which the stimulation is also synchronized with the user's exhalation but without the user's attention being necessary, since the stimulator detects the respiratory cycle and only stimulates during exhalation. In this way, the user can devote his attention to other activities because the stimulator is responsible for stimulating in sync with the breath.

Preferably, the electrical charge applied to each user in each stimulation can be personalized. Initially, each user is assigned an electrical charge depending on his/her profile, but based on the analysis of the data captured by the biosensor, the electrical charge to be injected can be customized. The stimulator keeps track of the applied electrical charge by stopping the stimulation when the set electric charge of the session has been reached. It is also possible to assign a maximum daily dose that the device will control not to be exceeded.

Typically, the device of the invention further comprises a smartphone application that allows the user to interact with the neurostimulator.

According to a second object, the invention relates to an auricular neurostimulation system comprising an auricular neurostimulation device and a charging case where the device can charge an internal battery and where the device discharges into this case the data captured by the photoplethysmographic sensor during stimulation and sends them to a dedicated platform in the cloud.

The cloud platform can also integrate data obtained from devices for continuous monitoring of cardiac activity such as watches, bracelets or rings, among others. The analysis of these data allows, for example, to know what the pattern of evolution of a user's stress is like and to define personalized stimulation treatments to prevent high peaks.

A method of operation of an auricular neurostimulation system, that is not claimed as part of the present application, comprises the following steps:
- set up step, where an electrical charge value (therapeutic dose) to apply in each stimulation session and a maximum daily electrical charge depending on each user;
- selection of the stimulation protocol (BEAT, BFS, EVANS) by the user;
- when the device is arranged on the user's ear, it starts with the stimulation, which continues until the electrical charge assigned to the session is reached or the maximum daily charge is reached;
- during stimulation, the biosensor stores readings of the user;
- once the stimulation is completed, the device downloads the session data into the charging case and the battery of the device is also recharged;
- the charging case sends the data from each stimulation session to the cloud, so the information can be properly analyzed;
- the platform in the cloud stores the data of the sessions sent to it;
- an algorithm analyses all the data to optimize the dose of electrical charge required by each user so it can be modified if desired.
- a further algorithm can send to the app a notification recommending stimulation sessions that prevent stress peaks based on data available on the cloud platform obtained from devices for continuous monitoring of cardiac activity.

### Brief description of the drawings

Further features, advantages and objects of the present invention will become apparent for a skilled person when reading the following detailed description of nonlimiting embodiments of the present invention, when taken in conjunction with the appended drawings, in which:
**FIG. 1A**: Detailed view of the different ear areas that may be stimulated in a human person.
**FIG. 1B****:**Neural anatomy of the ABVN and its connections to the different brain regions.
**FIG. 2**: Perspective view of the auricular neurostimulation device according to a first preferred embodiment of the present invention, showing its main components.
**FIG. 3**: Lateral perspective view of the auricular neurostimulation device according to a first preferred embodiment of the present invention, as represented in Figure 2.
**FIG. 4**: Perspective view of the auricular neurostimulation device according to a first preferred embodiment of the present invention, as represented in Figure 2, shown in the position where it will be placed in the ear of the patient.
**FIG. 5**: Perspective view of the auricular neurostimulation device according to a first preferred embodiment of the present invention, as represented in Figure 2, from its bottom position.
**FIG. 6**: Scheme with the components of the connected auricular neurostimulation system of the invention.
**FIG. 7**: Perspective view of the auricular neurostimulation device of the invention in an alternative mode of implementation to that in Figure 2, with the electronics located Behind The Ear (BTE).
**FIG. 8A****:**Graph showing the stimulation pattern of the auricular neurostimulation device of the present invention.
**FIG. 8B**:Graph showing the stimulation pattern of the auricular neurostimulation device of the present invention, synchronized with the patient's exhalation.
**FIG. 9**: Diagram of the electronic Printed Circuit Board (PCB) of the auricular neurostimulation device of the invention, where discontinuous lines represent flexible parts and continuous lines rigid parts.
**FIG. 10**: Graph showing the Vagus sensory evoked potential (VSEP) induced by auricular stimulation applied comparing the auricular neurostimulation device of the present invention with a prior art stimulation device.

### Detailed description of the exemplary embodiments

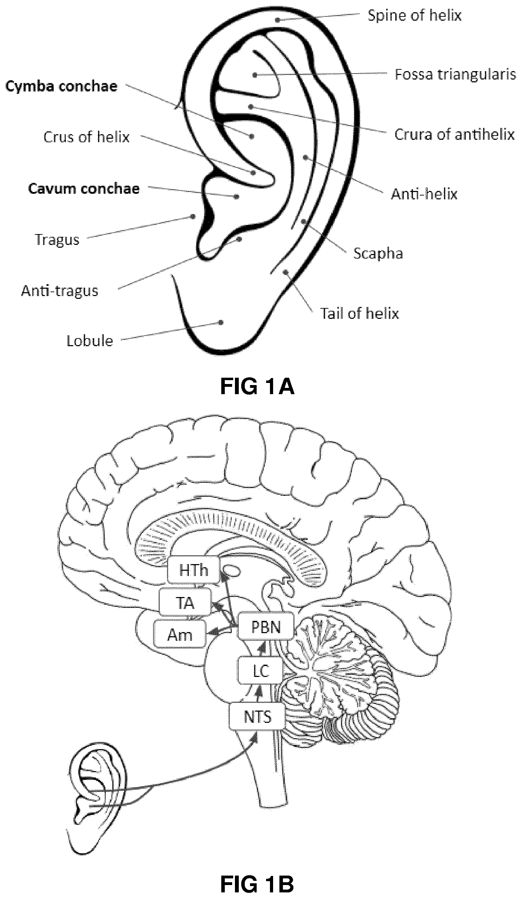

The object of the invention is a connected auricular neurostimulation device 1, wearable by a patient that optimizes the stimulation of the ABVN present in the cymba and cavum conchae, as it can be seen in Figure 1A.

Figure 1B shows the neural anatomy of the ABVN and its connections to the different brain regions. As depicted, the auricular branch of the vagus nerve (ABVN) projects to the nucleus tractus solitari (NTS), which is further connected with other brain regions, such as the locus coeruleus (LC), parabrachial nucleus (PBN), hypothalamus (Hth), nucleus accumbens (TA) and amygdala (Am).

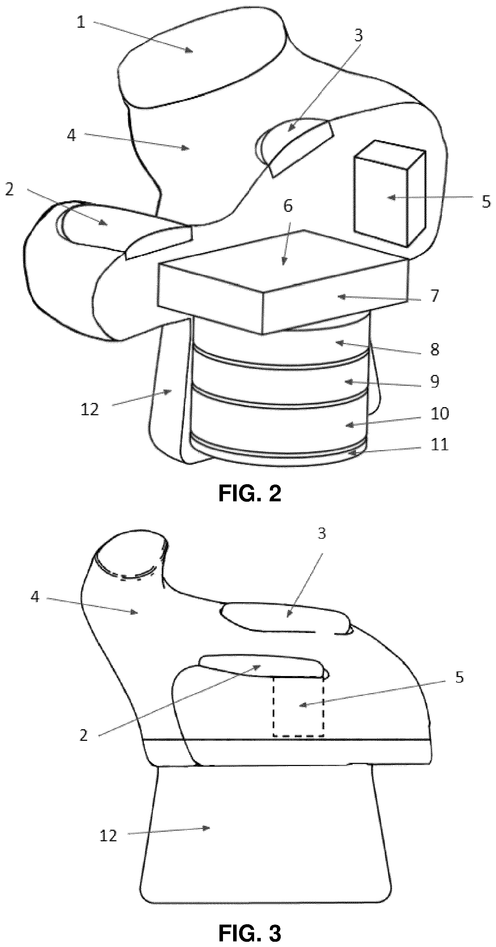

The auricular neurostimulation device 1 of the invention comprises the following components, as represented in Figure 2, according to a first preferred embodiment where the device 1 is arranged in the patient's ear:
- An electrode 2 that occupies the entire section of the cymba (the only ear zone with 100% ABVN). In a preferred embodiment, this electrode is configured as a working electrode on which a cathodic stimulation is applied in order to maximize the activation of the ABVN.
- An electrode 3 placed in the cavum conchae (ear zone with 45% ABVN). In a preferred embodiment, this electrode serves as a reference for applying the electrical voltage difference generated by cathodic stimulation.
- The electrodes are typically manufactured using graphene, biocompatible metals such as titanium, nickel titanium (nitinol), platinum, platinum-iridium, non-toxic metals as gold, conductive biocompatible inks for 3D printing or flexible conductive biocompatible polymers that adapt to the anatomy of the stimulation zone, therefore providing good comfort and perfect adaptation to the patient's ear.
- Earmold 4: This part is used as a support for the cymba and cavum conchae electrodes 2, 3 respectively in order to ensure that the positioning of these electrodes 2, 3 is adequate to maximize cymba and cavum conchae stimulation. The advantage of this earmold 4 in the device 1 of the invention is that it is custom made to the user's anatomy, so it can be personalized and shaped to optimally fit each patient's or user's anatomy. The earmold material is biocompatible and preferably thermoelastic so that it improves the fit with the user's ear as the earmold acquires body temperature.
- Photoplethysmographic or biosensor 5: This sensor serves to measure the environment temperature and the user's temperature and to estimate the amount of hemoglobin and oxyhemoglobin circulating through the most superficial capillary vessels of the ear of the patient or user, once the device 1 has been arranged on him or her. These data can be used to calculate the heart rate, the heart rate variability (HRV) and the oxygen saturation, among other biomedical variables and to detect the breathing phase (exhalation or inhalation) of the user or patient.

This photoplethysmographic or biosensor 5 is able to detect a very low heart rate of the user: in case this is detected, the device 1 is configured to automatically stop the stimulation.

In addition, the measurements made by the sensor make it possible to know how much electrical charge needs to be applied to each user at any given time to achieve vagal activation. This allows personalizing the stimulation treatments reaching efficiency levels much higher than other existing devices known in the art.

Moreover, the photoplethysmographic or biosensor 5 is also able to detect the breathing phase (exhalation or inhalation) of the patient or user wearing it with the aim to automatically synchronize the stimulation of the device 1 only with the user's exhalation with the goal to obtain a more efficient activation of the vagus nerve.
- Electronic circuit 6: The auricular neurostimulation device 1 is equipped with an electronic circuit 6 allowing it to develop the following functionalities, as indicated below.
   - Generation of stimulation patterns with variable duration, intensity, frequency of bursts and pulses, number of pulses per burst, pulse widths, and pulse delays, amongst others, by applying electrical voltage differences between electrode 2 and 3.
   - Generation of stimulation patterns synchronized with the exhalation of the user.
   - Control of electrical charge applied in each stimulation and daily-accumulated charge.
   - Exchange of data with external devices through wireless connections.
   - Data exchange with a charging case.
   - Wireless charging of the battery 10 by electromagnetic induction with a charging case.
- Faceplate 12: The auricular neurostimulation device 1 is equipped with a faceplate 12 that protects the electronic circuit 6 and makes it easy for the user to pick the device up for its placement and removal in the user's ear and in the charging case 13.

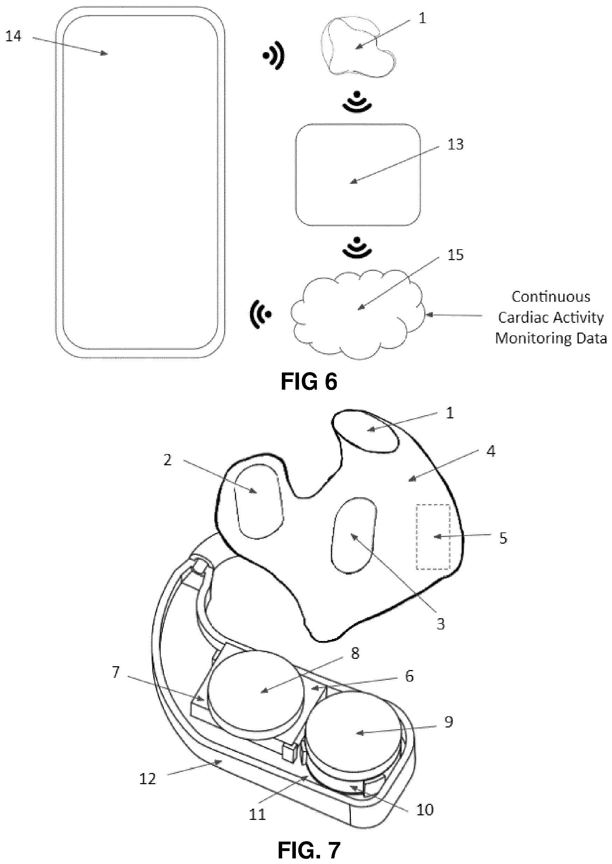

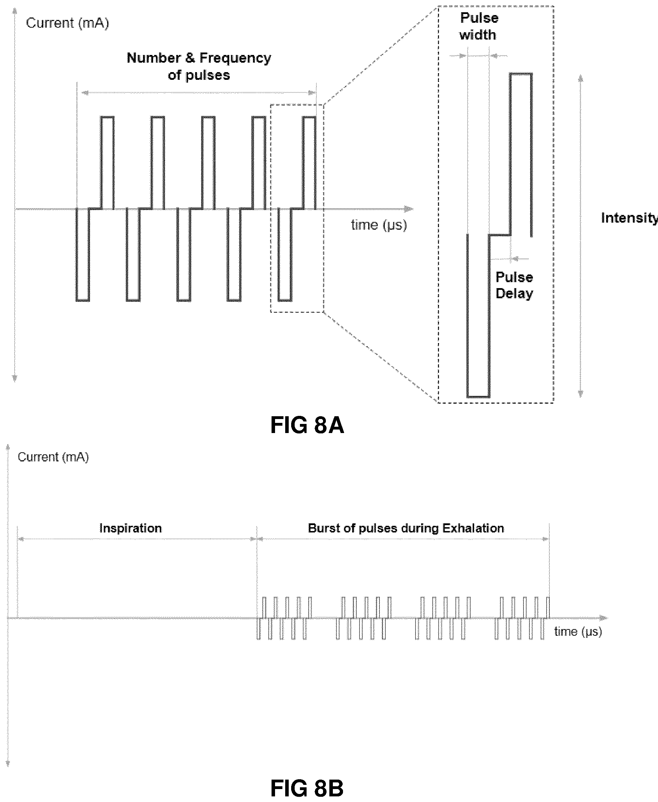

Moreover, an external charging case 13 and the connection of an internal application for smartphone 14 of the device 1 to an external cloud 15 configure a complete auricular neurostimulation system according to the invention, as shown in figure 6.
- Charging case 13: The auricular neurostimulation device 1 is stored when not used in a case 13 that charges its battery 10 wirelessly by electromagnetic induction. The device 1 discharges into this case 13 the data captured by the photoplethysmographic or biosensor 5 during stimulation when in use and sends them to a dedicated platform in the external cloud 15.
- Application for smartphone: The auricular neurostimulation device 1 has a smartphone application 14 that allows the patient or user to interact with the neurostimulation device 1, for example configuring certain stimulation parameters. The app 14 exchanges data with the dedicated platform in the cloud 15 to where the data captured by the photoplethysmographic or biosensor 5 during stimulation are sent.
- Stimulation protocols: The auricular neurostimulation device 1 implements a plurality of charge-controlled stimulation protocols. Preferably, these stimulation protocols are cathodic stimulation protocols. The charge is injected by applying an electrical voltage difference in the cymba electrode 2 with respect to the cavum electrode 3 that varies in real time depending on the impedance of the electrode-skin contact. The electrical voltage difference applied is a rectangular, biphasic, symmetrical wave with a delay between the first pulse and the second pulse (see Figure 8A). The first pulse of the waveform, or stimulating pulse, is used to elicit the desired physiological effect such as initiation of an action potential in the nerve endings, and the second pulse, or reversal pulse, is used to reverse electrochemical processes occurring during the stimulating pulse. The stimulation pulse is negative (cathodic stimulation) as it achieves faster depolarization of nerve endings than a positive pulse (anodic stimulation). It is estimated that the depolarization that occurs with an anodic stimulation is roughly one-seventh to one-third that of the depolarization with cathodic stimulation (Daniel R. Merrill et al. 2004). Thus, cathodic stimulation requires less current to bring an nerve ending to threshold. The addition of a delay between the stimulation and reversal pulses also contributes to the reduction of the threshold to achieve the action potential of the nerve endings. However, the delay should not be too long to prevent the products of the Faradaic reactions caused by the stimulation pulse from accumulating to levels that could cause tissue damage. Delay values between 0 and 150µs are considered appropriate.

Stimulation protocols include pulse bursts as these improve the effectiveness of stimulation. The action potentials triggered at the sensory auricular vagus endings in response to continuous stimuli are less likely to influence systemic regulation or brain activity, rather than a rhythmic sequence of these impulses. This is because gradual natural sensory information is coded as the gradual temporal density of non-gradual impulses, likewise, coded as the instantaneous frequency of impulses. On the other hand, the brain with its very large number of neurons and its sophisticated processing is not likely to respond reasonably to a single or a few impulses but to a train of impulses. Stimulation protocols may include between 1 and 10 burst per second.

The intensity of the electric current, the width of the pulses and their frequency are also variable in the stimulation protocols. The stimulation intensity can vary between 0 and 5mA as it has been proven experimentally that in this range is sufficient to produce an effective stimulation of nerve endings in a way that is comfortable for the user. The pulse width usually determines the type of fibers to be excited. That is, short pulses recruit easily excitable thick fibers only while elongated pulses recruit both thick and thin fibers. The ABVN is composed mainly of fibres Aβ, Aδ and C (Safi et al., 2016). The Aβ have diameters between 5 and 12µm and are associated with sensitive functions. The Aδ has diameters between 3 and 6µm and transmits localized pain, temperature and touch. Those of type C have diameters between 0,4 and 1,2µm and transmit diffuse pain and temperature. In stimulation, it is desirable to activate Aβ and not Aδ or C, so the stimulation pulse must be short. It has been proven that values between 50 and 250µs can be appropriate. Another important parameter in stimulation is the frequency or number of pulses per second, since depending on its value, one type of fiber or another is activated. The range of frequency variation in the stimulation protocols is between 1 and 30 Hz.

The different stimulation protocols can be conceptually grouped into three modalities:
- Protocols BEAT in which the patient's breathing is not taken into account.
- Protocols BFS that establish guidelines for the user to accommodate his breathing rhythm.
- Protocols EVANS in which the device automatically detects the user's inspiration and exhalation and stimulates only during exhalation.

BEAT type protocols apply pulse burst with variable parameters within the above ranges (see Figure 8A). The BSF and EVANS protocols also apply pulse burst with variable parameters but only during user expiration (See Figure 8B). In the BSF the user adapts his exhalation to the moments of stimulation and in the EVANS the device detects the exhalation and synchronizes the stimulation with it.

The auricular neurostimulation device 1 of the invention is configured to detect when the user is exhaling thanks to the photoplethysmographic or biosensor 5. The duration of the stimulation sessions depends on the electrical charge (dosis) assigned to the session and the stimulation intensity selected by the user. Initially, each user is assigned an electrical charge depending on his/her profile, but based on the analysis of the data captured by the biosensor 5, the electrical charge to be applied can be customized. The stimulator keeps track of the applied electrical charge by stopping the stimulation when the set electric charge of the session has been reached. It is also possible to assign a maximum daily dose that the device will control not to be exceeded.

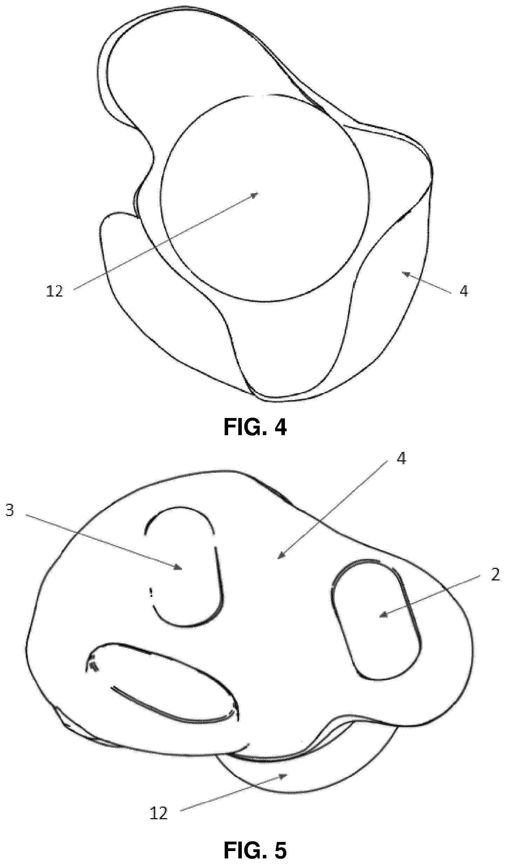

The auricular neurostimulation device 1 of the invention has been described according to a preferred embodiment, as represented in Figures 2-5: according to this embodiment, the electronic of the device 1 is placed in the conchae of the user's ear (ITE or In The Ear). However, a different possible embodiment of the device 1 of the invention would be to configure the device to be placed behind the ear (BTE) of the user, as represented in Figure 7. The components of the device are the same as in the preferred configuration (that represented in Figures 2-5) but having a different configuration allowing the device to be placed behind the ear of the user. This way, the electronic components of the device are arranged behind the user's ear and are not visible from outside. Moreover, the user is very comfortable with this BTE configuration.

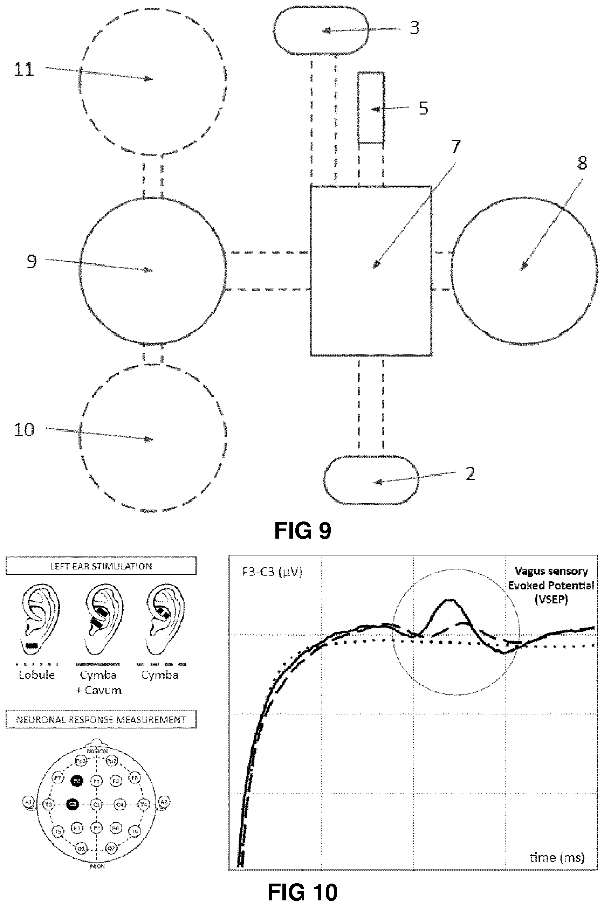

The electronic circuit 6 in the device of the invention is built on a Printed Circuit Board (PCB) that combines rigid parts with flexible parts, as shown in Figure 9. The parts can be stacked to form an assembly that can be inserted into the faceplate 12, both in the ITE (In The Ear) configuration of Figures 2-5 and in the BTE (Behind The Ear) configuration of Figure 7. The electronic circuit 6 comprises the following elements:
- A central circuit 7 that controls all the functioning of the device, including the communication with the smartphone and the charger case, the generation of the stimulation patterns and the adjustment of the electrical voltage difference applied to the electrodes 2 and 3, according to the skin-electrode contact impedance.
- A voltage amplifier 8 that raises the voltage supplied by the battery to the level necessary to apply the electrical voltage difference to electrodes 2 and 3 required at any given time.
- A charger circuit 9 that takes advantage of the electric current generated in the coil 11.
- A battery 10 that is rechargeable with the current generated in coil 11.
- A coil 11 that receives the magnetic field created by a coil located in the charger case and generates electric current to recharge the battery.

Figure 10 shows the Vagus sensory evoked potential (VSEP) induced by auricular stimulation applied a) in the lobe where there are no vagal nerve endings b) according to the electrode arrangement of the object of the present invention with two large electrodes, one in cymba as working electrode and one in cavum as counter electrode c) according to the electrode arrangement of Cerbomed's stimulator (corresponds with prior art device disclosed in EP3100764) with two small electrodes in cymba. Vagus sensory evoked potential (VSEP) via electrical ABVN stimulation and to measure it with EEG electrodes on the scalp as a far field potential has been demonstrated to be another way to evaluate the vagus nerve response (Fallgatter AJ et al, 2003,Lewine JD. et al., 2019). The graphs have been obtained by measuring neuronal electrical activity between points C3-F3 of the international 10-20 EEG measurement system. Neuronal activity is produced by the postsynaptic potentials generated in the nucleus of the solitary tract (NTS) in response to auricular electrical stimulation (See Figure 1B). The graphs shown have been obtained by averaging the neuronal response (vagal sensory evoked potential-VSEP) after the application of at least 50 electrical stimulation pulses. The amplitude of the VSEP is representative of the effectiveness of the stimulation. The results obtained in the measurement of 12 volunteers indicate that the stimulation performed as indicated in the present invention generates a vagal evoked potential between 3 and 7 times higher than that generated by the Cerbomed stimulation (prior art).

The auricular neurostimulation device 1 of the invention presents as explained below, several advantages with respect to other neurostimulation devices known in the state of the art, in particular relating to safety, effectiveness, comfort, usability and personalization:
Safety. The photoplethysmographic or biosensor 5 included in stimulator 1 makes it possible to anticipate a very low heart rate and stop the stimulation to prevent risky situations. On the other hand, circuit 6 keeps track of the daily electric charge introduced to the user, preventing it from exceeding a limit. It also adjusts in real time the electrical voltage difference applied to electrodes 2 and 3 according to the impedance of the contact of the electrodes with the skin, preventing the applied current from rising to dangerous limits if the impedance drops quickly, due to effects such as electroporation.
Effectiveness. The auricular neurostimulation device 1 of the invention applies cathodic stimulation in the cymba which is the only area of the ear in which 100% of the nerve endings are vagal. Of all the auricular stimulators found in this state of the art, only Cerbomed stimulate in the cymba. However, it has been proven in the studies carried out with vagus sensory evoked potentials (See Figure 10) that the neuronal response with the electrode arrangement as described in this invention is between 3 and 7 times more effective than the response with Cerbomed's electrode arrangement (prior art).

The reasons are as follows:
- Electrode 2 has a larger surface area (typically between 15 and 45 mm²) than Cerbomed's electrodes (prior art). So it stimulates a larger area.
- The earmold that positions the electrodes is customized to the geometry of the user's ear so the quality of contact is much better.
- Cathodic stimulation (the stimulation pulse is negative) is used on the cymba, which is 3 to 7 times more effective than the anodic stimulation (positive stimulation pulse) used by Cerbomed according to prior art patent PCT/EP2015/001279.
- A delay between the stimulation pulse and the reversal pulse is included, which also increases the effectiveness of the stimulation.
- A second large-surface electrode is included in the cavum conchae, an area with 45% vagal nerve endings, which also contributes to stimulation.

In addition, the device 1 can execute BSF or EVAN protocols, in which stimulation is synchronized with the user's exhalation, thus enhancing parasympathetic activation. During exhalation, the activation of arterial baroreceptors leads to the excitation of second-order neurons of the Nucleus Tractus Solitarii (NTS) that in turn increases premotor cardiovagal neuron firing rate. In addition, during inhalation, NTS receives inhibitory inputs from ventral respiratory nuclei in the medulla, reducing vagal outflow to the heart that could lead to respiratory sinus arrhythmia (RSA). As the dorsal medullary vagal system operates in tune with respiration, gating vagal afferent stimulation to the exhalation phase of respiration optimizes ABVN stimulation and its effects on cardiac vagal modulation.

Comfort. Transcutaneous electrical stimulation generates a throbbing sensation when the current density (amount of electrical current per unit area) is too high. The way to avoid this unpleasant sensation is to apply the electric current evenly over a large contact surface. To achieve this, device 1 of the invention uses large surface electrodes which are also placed in earmold 4 whose geometry is customized for each user. In this way, the contact zone between the electrodes and the stimulation zones is wide and of good quality so that the current can flow without concentrating excessively at any point.

Usability. The majority of auricular neurostimulators known in the state of the art consist of a large generator to which an accessory that applies an electrical voltage difference to some parts of the ear is connected by means of a cable. The volume and weight of the set limits its portability and consequently its availability for use. The auricular neurostimulation device 1 of the present invention, however, has been developed as a small, lightweight device that is comfortable to wear. Moreover, being configured as a wireless earbud, the user recognizes it as a familiar product so the adoption of use is very simple.

Personalization. The auricular neurostimulation device 1 of the invention includes two types of customizations: anatomical and therapeutic, as they will be explained in what follows.
- *Anatomical customization* consists of customizing the shape that is in contact with the user's ear. To achieve this, a sample is taken from the user's ear and then scanned. Another option is to scan the user's ear directly in 3D. The 3D geometry generated by both options is used to create a custom earmold 4 to which the faceplate 12 is added with the electronic circuit 6, and the stimulating device 1 is manufactured.
- *Therapeutic personalization* consists of personalizing the electrical charge (dosis) introduced by the stimulation and the possibility of stimulating it in a synchronized manner together with the user's breathing (EVANS/BFSs protocol).

In addition, if the user integrates data from devices for continuous monitoring of cardiac activity such as watches, bracelets or rings, among others, the analysis of these data allows, for example, to know the pattern of evolution of stress of a user and define personalized stimulation treatments to prevent high peaks.

The auricular neurostimulation device 1 of the invention is a connected device, the connection of it being made through two pathways. On the one hand, it can connect wirelessly (e.g. via Bluetooth) to an application for a smartphone, which in turn is connected, to a software in the cloud. On the other hand, the charging case 13 is connected to the software in the cloud, in order to be able to transmit the stimulator usage data, including those captured by the photoplethysmographic or biosensor 5.

In a broader way, as described earlier, the invention further relates to an auricular neurostimulation system comprising an auricular neurostimulation device 1 as described, an external charging case 13, and the connection of an internal application in the device to a cloud software for its correct connection and parametrization.

The method of operation of the auricular neurostimulation system, which is not claimed as part of the present invention, comprises several steps, which are now described in detail:

### First step: Setup.

1. The user creates an account on the system using the application for the smartphone or the web. For the registration, the user is asked for a series of personal data. The system assigns an initial electrical charge value (therapeutic dose) to apply in each stimulation session and a maximum daily electrical charge, depending on the user's profile. The initial values of the stimulation electric charge and the maximum daily electric charge are assigned on the basis of statistical studies. However, as the stimulator is used, the analysis of the data captured by photopletismographic or biosensor 5 allows these values to be customized.
2. Once the registration is complete, the user logs into the application and connects to the device 1 to match its serial number to the user account, so the device 1 is associated to the user.
3. The application asks the user to set his/her *'perception and pain thresholds'* (this can be changed at a later stage). The pain threshold indicates that Aβ type fibres have been stimulated and Aδ and C type fibres are beginning to be stimulated. With the value of both thresholds, the application establishes the range in which the stimulation intensity must be placed, so that the stimulation is effective but also comfortable. The user can now choose the stimulation intensity that is most comfortable within the range set by the app. This value of intensity can be modified whenever the user wishes, but always within the range established by the app.

### Second Step: Stimulation.

1. The user selects a stimulation protocol from those available (BEAT, BFS or EVANS types). The app sends the data of the protocol selected to the device 1. From here on, the stimulator can operate autonomously without the need to be connected to the smartphone. This will only be necessary if the user want to change the stimulation protocol or the intensity of stimulation.
2. When the user takes the device 1 out of the charging case 13, the device detects it thanks to a magnetic switch connecting it to the case 13, so it is then activated. It also detects when it is in the ear thanks to the proximity detector of the photopletismographic or biosensor 5. If the stimulator is well placed in the user's ear and therefore the impedance of contact of the electrodes with the stimulation zones is good, the device 1 starts to stimulate automatically according to defined stimulation conditions.
3. The device 1 keeps track of the electrical charge it is inserting in the user's ear. When it reaches the assigned value (therapeutic dose), it stops automatically. It also stops when it has reached the maximum daily limit or when the user removes the device (it is detected by the proximity detector of the photoplethysmographic sensor 5).
4. During stimulation, the photoplethysmographic or biosensor 5 stores temperature, hemoglobin and oxyhemoglobin readings of the user.
5. Once the stimulation is completed, the device 1 downloads the session data (day and time, stimulation time, stimulation parameters and biosensor data) into the charging case 13 and prepares for the next session. The battery 10 of the device 1 is also recharged wirelessly in the case 13.
6. The charging case 13 sends the data from each stimulation session to the cloud, so the information can be properly analyzed.
7. The platform in the cloud stores the data of the sessions sent to it.
8. An algorithm analyses all the data to optimize the dose of electrical charge required by each user. If it is decided to change it, the platform sends the new values to the application so that the next stimulation is done with these newly modified values.
9. If the user has data available on the cloud platform obtained from devices for continuous monitoring of cardiac activity such as watches, bracelets or rings, among others, a further algorithm can send to the app a notification recommending stimulation sessions that prevent stress peaks.

Although the present invention has been described with reference to preferred embodiments thereof, many modifications and alterations may be made by a person having ordinary skill in the art without departing from the scope of this invention, which is defined by the appended claims.

## Claims

1. Auricular neurostimulation device (1) wearable by a user and configured to stimulate the Auricular Branch of Vagus Nerve (ABVN) on the user's ear, wherein
the device (1) comprises at least two electrodes (2, 3) designed to be located in the cymba and in the cavity of the cavum conchae respectively;
the electrodes (2, 3) electrically stimulate the nerve ramifications of the cymba and the cavum conchae respectively;
**characterized in that**
the device (1) is configured as a wireless earbud comprising an earmold (4) and a miniaturized faceplate (12) wherein,
the electrodes (2, 3) are arranged on the earmold (4) which is customized to the user's ear anatomy such that the cymba electrode (2) is a large-surface electrode covering almost the whole cymba surface area of the user's ear.
a photoplethysmographic or biosensor (5) with a miniaturized configuration is arranged inside the earmold (4),
the miniaturized faceplate (12) incorporates inside it all the elements of an electronic circuit (6) built on a Printed Circuit Board (PCB) that combines rigid elements with flexible elements, both in an ITE (In The Ear) configuration or in an BTE (Behind The Ear) configuration.

2. Auricular neurostimulation device (1) according to claim 1 wherein the electrodes (2, 3) are made of graphene, biocompatible metals, non-toxic metals, conductive biocompatible inks for 3D printing or flexible conductive biocompatible polymers, and wherein the earmold (4) is made of a biocompatible material and preferably thermoelastic so that it improves the fit with the user's ear as the earmold acquires body temperature.

3. Auricular neurostimulation device (1) according to claim 1,
wherein the photoplethysmographic or biosensor (5) measures the environment temperature and the user's temperature and to estimate the amount of hemoglobin and oxyhemoglobin circulating through the most superficial capillary vessels of the ear of the patient or user, these data being used to calculate the heart rate, the heart rate variability (HRV) and the oxygen saturation and to detect the breathing phase (exhalation or inhalation) of the user or patient;
and wherein the measurements made by the photoplethysmographic or biosensor (5) are used to determine which electrical charge is the most suitable for each user at any given time.

4. Auricular neurostimulation device (1) according to claim 1, wherein the electronic circuit (6) comprises the following elements:
• a central circuit (7) that controls all the functioning of the device (1),
• a voltage amplifier (8) that raises the voltage supplied by a battery (10),
• a charger circuit (9) that takes advantage of the electric current generated in a coil (11) that receives the magnetic field created by a further coil located in a charger case.
• a battery (10) that is rechargeable with the current generated in coil (11).

5. Auricular neurostimulation device (1) according to claims 1 - 4 , wherein the electronic circuit (6) is configured to:
• Generate stimulation patterns with variable duration, intensity, frequency of bursts and pulses, number of pulses per burst, pulse widths, and pulse delays, amongst others.
• Generate stimulation patterns synchronized with the exhalation of the user.
• Control the electrical charge applied in each stimulation and daily-accumulated charge.
• Exchange data with external devices through wireless connections.
• Exchange data with a charging case (13).
• Wireless charging of a battery (10) by electromagnetic induction with a charging case.

6. Auricular neurostimulation device (1) according to any of any of the previous claims, wherein the stimulation done by it, is synchronized with the exhalation-breathing phase of the user.

7. Auricular neurostimulation device (1) according to any of any of the previous claims,
**characterized in that** it implements a plurality of stimulation protocols, where the intensity of the electric current, pulse width and repetition frequency of the pulses is variable;
and wherein the stimulation protocols are based on a waveform of rectangular, biphasic, symmetric and with a delay between the negative and positive pulse.

8. Auricular neurostimulation device (1) according to claims 6-7 wherein the stimulation protocol is any of the following:
• a BEAT type protocol, consisting of the continuous application of burst of pulses.
• a BFS (Breathing Focused on Stimulation) type protocol combining stimulation moments with standstill moments, so the user breathes in during the stop time and breathes out during the stimulation.
• an EVANS (Exhalation Vagus Auricular Nerve Stimulation) type protocol stimulating only during the exhalation of the user.

9. Auricular neurostimulation device (1) according to any of the previous claims wherein the electrical charge quantity applied to electrodes (2, 3) is personalized for each user, according to the therapeutic dose needed.

10. Auricular neurostimulation device (1) according to any of the previous claims wherein the electrical voltage difference applied to electrodes (2 and 3) is adjusted in real time to the impedance of the contact of the electrodes with the skin, in order to ensure that the intensity of stimulation is as established.

11. Auricular neurostimulation system comprising an auricular neurostimulation device (1) according to any of claims 1-10 and a charging case (13) where the device (1) is stored when not used in order to charge its battery (10) wirelessly by electromagnetic induction and where the device (1) discharges into this case (13) the data captured by the photoplethysmographic or biosensor (5) during stimulation and sends them to a dedicated platform in the cloud (15).

12. Auricular neurostimulation system comprising an auricular neurostimulation device (1) according to claim 11 further comprising a smartphone application that allows the user to interact with the neurostimulation device 1.

## Patentansprüche

1. Aurikuläre Stimulationsvorrichtung (1), die von einem Benutzer getragen werden kann und dafür ausgelegt ist, den Aurikularast des Vagusnervs (Auricular Branch of Vagus Nerve, ABVN) am Ohr des Benutzers zu stimulieren, wobei
die Vorrichtung (1) wenigstens zwei Elektroden (2, 3) umfasst, die dazu bestimmt sind, in der Ohrmuschel (Cymba) bzw. in der Höhle des Gehörgangseingangstrichters (Cavum conchae) angeordnet zu werden;
die Elektroden (2, 3) elektrisch die Nervenverzweigungen der Cymba bzw. des Cavum conchae stimulieren;
**dadurch gekennzeichnet, dass**
die Vorrichtung (1) als drahtloser Ohrstecker ausgestaltet ist, der ein Ohrpassstück (4) und eine miniaturisierte Frontplatte (12) umfasst, wobei,
die Elektroden (2, 3) auf dem Ohrpassstück (4) angeordnet sind, das an die Ohranatomie des Benutzers angepasst ist, so dass die Cymba-Elektrode (2) eine großflächige Elektrode ist, die fast die gesamte Oberfläche der Cymba des Ohrs des Benutzers abdeckt,
ein photoplethysmographischer oder Biosensor (5) mit einer miniaturisierten Ausgestaltung im Inneren des Ohrpassstücks (4) angeordnet ist,
die miniaturisierte Frontplatte (12) darin alle Elemente einer elektronischen Schaltung (6) enthält, die auf einer gedruckten Schaltung (PCB, Printed Circuit Board) aufgebaut ist, die starre Elemente mit flexiblen Elementen kombiniert, sowohl in einer IdO (Im-Ohr)-Konfiguration als auch in einer HdO (Hinter-dem-Ohr)-Konfiguration.

2. Aurikuläre Neurostimulationsvorrichtung (1) gemäß Anspruch 1, wobei die Elektroden (2, 3) aus Graphen, biokompatiblen Metallen, nichttoxischen Metallen, leitfähigen biokompatiblen Tinten für den 3D-Druck oder flexiblen leitfähigen biokompatiblen Polymeren bestehen, und wobei das Ohrpassstück (4) aus einem biokompatiblen und vorzugsweise thermoelastischen Material hergestellt ist, so dass die Passform mit dem Ohr des Benutzers verbessert wird, wenn das Ohrpassstück Körpertemperatur annimmt.

3. Aurikuläre Neurostimulationsvorrichtung (1) gemäß Anspruch 1,
wobei der photoplethysmographische oder Biosensor (5) die Umgebungstemperatur und die Temperatur des Benutzers misst und die Menge an Hämoglobin und Oxyhämoglobin schätzt, die durch die oberflächlichsten Kapillargefäße des Ohrs des Patienten oder Benutzers zirkuliert, wobei diese Daten zum Berechnen der Herzfrequenz, der Herzfrequenzvariabilität (HRV, Heart Rate Variability) und der Sauerstoffsättigung und zum Erkennen der Atemphase (Ausatmung oder Einatmung) des Benutzers oder Patienten verwendet werden;
und wobei die vom photoplethysmographischen oder Biosensor (5) durchgeführten Messungen dazu dienen zu bestimmen, welche elektrische Ladung für den jeweiligen Benutzer zu einem gegebenen Zeitpunkt am besten geeignet ist.

4. Aurikuläre Neurostimulationsvorrichtung (1) gemäß Anspruch 1, wobei die elektronische Schaltung (6) die folgenden Elemente umfasst:
• eine zentrale Schaltung (7), die alle Funktionen der Vorrichtung (1) steuert,
• einen Spannungsverstärker (8), der die von der Batterie (10) gelieferte Spannung anhebt,
• eine Ladeschaltung (9), die den elektrischen Strom nutzt, der in einer Spule (11) erzeugt wird, die das Magnetfeld aufnimmt, das von einer weiteren Spule in einem Ladegehäuse erzeugt wird,
• eine Batterie (10), die mit dem in der Spule (11) erzeugten Strom aufgeladen werden kann.

5. Aurikuläre Neurostimulationsvorrichtung (1) gemäß einem der Ansprüche 1-4, wobei die elektronische Schaltung ausgelegt (6) ist zum:
• Erzeugen von Stimulationsmustern mit variabler Dauer, Intensität, Frequenz von Bursts und Impulsen, Anzahl der Impulse pro Burst, Impulsbreiten und Impulsverzögerungen u. a.,
• Erzeugen von Stimulationsmustern, die mit der Ausatmung des Benutzers synchronisiert sind,
• Steuern der bei jeder Stimulation angelegten elektrischen Ladung und der täglich akkumulierten Ladung,
• Austauschen von Daten mit externen Vorrichtungen über drahtlose Verbindungen,
• Austauschen von Daten mit einem Ladegehäuse (13),
• drahtlosen Laden einer Batterie (10) durch elektromagnetische Induktion in einem Ladegehäuse.

6. Aurikuläre Neurostimulationsvorrichtung (1) gemäß einem der vorstehenden Ansprüche, wobei die von dieser ausgehende Stimulation mit der Ausatmungsphase des Benutzers synchronisiert ist.

7. Aurikuläre Neurostimulationsvorrichtung (1) gemäß einem der vorstehenden Ansprüche, **dadurch gekennzeichnet, dass** sie mehrere Stimulationsprotokolle implementiert, wobei die Intensität des elektrischen Stroms, die Impulsbreite und die Wiederholungsfrequenz der Impulse variabel sind;
und wobei die Stimulationsprotokolle auf einer rechteckigen, zweiphasigen, symmetrischen Wellenform und mit einer Verzögerung zwischen dem negativen und dem positiven Impuls basieren.

8. Aurikuläre Neurostimulationsvorrichtung (1) gemäß Anspruch 6-7, wobei das Stimulationsprotokoll eines der folgenden ist:
• ein Protokoll vom Typ BEAT, das aus der kontinuierlichen Anwendung von Impulsbursts besteht,
• ein Protokoll des Typs BFS (Breathing Focused on Stimulation, Atmung fokussiert auf Stimulation), bei dem Stimulationsphasen mit Stillstandsphasen kombiniert werden, so dass der Benutzer während der Stillstandszeit einatmet und während der Stimulation ausatmet,
• ein Protokoll vom Typ EVANS (Exhalation Vagus Auricular Nerve Stimulation, Vagus-Aurikularnerv-Stimulation bei Ausatmen), das nur während der Ausatmung des Benutzers stimuliert.

9. Aurikuläre Neurostimulationsvorrichtung (1) gemäß einem der vorstehenden Ansprüche, wobei die elektrische Ladungsmenge, die an die Elektroden (2, 3) angelegt wird, für jeden Benutzer entsprechend der benötigten therapeutischen Dosis personalisiert ist.

10. Aurikuläre Neurostimulationsvorrichtung (1) gemäß einem der vorstehenden Ansprüche, wobei die elektrische Spannungsdifferenz, die an die Elektroden (2 und 3) angelegt wird, in Echtzeit an die Impedanz des Kontakts der Elektroden mit der Haut angepasst wird, um sicherzustellen, dass die Intensität der Stimulation wie festgelegt ist.

11. Aurikuläres Neurostimulationssystem, das eine aurikuläre Stimulationsvorrichtung (1) gemäß einem der Ansprüche 1-10 und ein Ladegehäuse (13) umfasst, wo die Vorrichtung (1) bei Nichtgebrauch aufbewahrt wird, um ihre Batterie (10) drahtlos durch elektromagnetische Induktion aufzuladen, und wo die Vorrichtung (1) die Daten, die vom photoplethysmographischen oder Biosensor (5) während der Stimulation erfasst werden, in dieses Gehäuse (13) abgibt und sie an eine spezielle Plattform in der Cloud (15) sendet.

12. Aurikuläres Neurostimulationssystem, das eine aurikuläre Stimulationsvorrichtung (1) gemäß Anspruch 11 umfasst, die ferner eine Smartphone-Anwendung umfasst, welche dem Benutzer ermöglicht, mit der Neurostimulationsvorrichtung 1 zu interagierten.

## Revendications

1. Dispositif de neurostimulation auriculaire (1) pouvant être porté par un utilisateur et configuré pour stimuler le rameau auriculaire du nerf vague (ABVN) sur l'oreille de l'utilisateur, dans lequel
le dispositif (1) comprend au moins deux électrodes (2, 3) conçues pour être situées dans la cymba et dans la cavité de la conque respectivement ;
les électrodes (2, 3) stimulent électriquement les ramifications nerveuses de la cymba et de la cavité de la conque respectivement ;
**caractérisé en ce que**
le dispositif (1) est configuré sous la forme d'une oreillette sans fil comprenant un embout auriculaire (4) et une face avant miniaturisée (12) dans lequel
les électrodes (2, 3) sont disposées sur l'embout auriculaire (4) qui est personnalisé selon l'anatomie de l'oreille de l'utilisateur de sorte que l'électrode (2) de cymba soit une électrode à grande surface recouvrant presque toute la surface spécifique de la cymba de l'oreille de l'utilisateur,
un photopléthysmographe ou biocapteur (5) ayant une configuration miniaturisée est disposé à l'intérieur de l'embout auriculaire (4),
la face avant miniaturisée (12) intègre en son sein tous les éléments d'un circuit électronique (6) construit sur une carte de circuit imprimé (PCB) qui combine éléments rigides et éléments souples, à la fois dans une configuration ITE (intra-auriculaire) ou dans une configuration BTE (contour d'oreille).

2. Dispositif de neurostimulation auriculaire (1) selon la revendication 1 dans lequel les électrodes (2, 3) sont constituées de graphène, de métaux biocompatibles, de métaux non toxiques, d'encres biocompatibles conductrices pour impression 3D ou de polymères biocompatibles conducteurs souples, et dans lequel l'embout auriculaire (4) est constitué d'un matériau biocompatible et de préférence thermoélastique de sorte qu'il améliore l'ajustement sur l'oreille de l'utilisateur à mesure que l'embout auriculaire atteint la température corporelle.

3. Dispositif de neurostimulation auriculaire (1) selon la revendication 1,
dans lequel le photopléthysmographe ou le biocapteur (5) mesure la température environnante et la température de l'utilisateur et permet d'estimer la quantité d'hémoglobine et d'oxyhémoglobine circulant par les vaisseaux capillaires les plus superficiels de l'oreille du patient ou de l'utilisateur, ces données étant utilisées pour calculer la fréquence cardiaque, la variabilité de la fréquence cardiaque (VFC) et la saturation en oxygène et pour détecter la phase respiratoire (expiration ou inspiration) de l'utilisateur ou du patient ;
et dans lequel les mesures faites par le photopléthysmographe ou le biocapteur (5) sont utilisées pour déterminer la charge électrique la plus adaptée à chaque utilisateur, à tout moment.

4. Dispositif de neurostimulation auriculaire (1) selon la revendication 1, dans lequel le circuit électronique (6) comprend les éléments suivants :
• un circuit central (7) qui commande tout le fonctionnement du dispositif (1),
• un amplificateur de tension (8) qui augmente la tension fournie par une batterie (10),
• un circuit (9) de chargeur qui bénéficie du courant électrique produit dans une bobine (11) qui reçoit le champ magnétique créé par une bobine supplémentaire située dans un étui chargeur,
• une batterie (10) qui est rechargeable avec le courant produit dans la bobine (11).

5. Dispositif de neurostimulation auriculaire (1) selon les revendications 1 à 4, dans lequel le circuit électronique (6) est configuré pour :
• générer des motifs de stimulation avec une durée, une intensité, une fréquence de salves et d'impulsions, un nombre d'impulsions par salve, des largeurs d'impulsion et des retards d'impulsion variables, entre autres choses,
• générer des motifs de stimulation synchronisés avec l'expiration de l'utilisateur,
• commander la charge électrique appliquée dans chaque stimulation et la charge accumulée quotidiennement,
• échanger des données avec des dispositifs externes au moyen de connexions sans fil,
• échanger des données avec un étui de charge (13),
• charger sans fil une batterie (10) par induction électromagnétique avec un étui de charge.

6. Dispositif de neurostimulation auriculaire (1) selon l'une quelconque des revendications précédentes, dans lequel la stimulation faite par celui-ci est synchronisée avec la phase respiratoire d'expiration de l'utilisateur.

7. Dispositif de neurostimulation auriculaire (1) selon l'une quelconque des revendications précédentes, **caractérisé en ce qu'**il met en œuvre une pluralité de protocoles de stimulation, où l'intensité du courant électrique, la largeur d'impulsion et la fréquence de répétition des impulsions sont variables ;
et dans lequel les protocoles de stimulation sont basés sur une forme d'onde rectangulaire, biphasée, symétrique et avec un retard entre l'impulsion négative et l'impulsion positive.

8. Dispositif de neurostimulation auriculaire (1) selon les revendications 6 et 7 dans lequel le protocole de stimulation est un élément quelconque parmi :
• un protocole de type BEAT, consistant en l'application continue d'une salve d'impulsions,
• un protocole de type BFS (respiration focalisée sur la stimulation) associant des moments de stimulation à des moments d'arrêt, de sorte que l'utilisateur inspire pendant le temps d'arrêt et expire pendant la stimulation,
• un protocole de type EVANS (stimulation du rameau auriculaire du nerf vague à l'expiration) stimulant uniquement pendant l'expiration de l'utilisateur.

9. Dispositif de neurostimulation auriculaire (1) selon l'une quelconque des revendications précédentes dans lequel la quantité de charge électrique appliquée à des électrodes (2, 3) est personnalisée pour chaque utilisateur, en fonction de la dose thérapeutique nécessaire.

10. Dispositif de neurostimulation auriculaire (1) selon l'une quelconque des revendications précédentes dans lequel la différence de tension électrique appliquée à des électrodes (2 et 3) est ajustée en temps réel à l'impédance du contact des électrodes avec la peau, dans le but de garantir que l'intensité de la stimulation est telle qu'établie.

11. Système de neurostimulation auriculaire comprenant un dispositif de neurostimulation auriculaire (1) selon l'une quelconque des revendications 1 à 10 et un étui de charge (13) où est rangé le dispositif (1) quand il n'est pas utilisé dans le but de charger sa batterie (10) d'une manière sans fil par induction électromagnétique et où le dispositif (1) décharge dans cet étui (13) les données capturées par le photopléthysmographe ou le biocapteur (5) pendant la stimulation et les envoie à une plateforme dédiée dans le nuage (15).

12. Système de neurostimulation auriculaire comprenant un dispositif de neurostimulation auriculaire (1) selon la revendication 11 comprenant en outre une application de téléphone intelligent qui permet que l'utilisateur interagisse avec le dispositif de neurostimulation 1.
